# EUROPÄISCHE PATENTSCHRIFT

(11) **EP 1 064 924 B1**
(45) Veröffentlichungstag und Bekanntmachung des Hinweises auf die Patenterteilung: **26.04.2006**
(21) Anmeldenummer: 00113725.6
(22) Anmeldetag: 28.06.2000
(51) Int. Cl.: A61K 8/81, A61Q 19/00, A61Q 19/10, C08F 226/00

(54) **Verwendung von vernetzten kationischen Polymeren in hautkosmetischen und dermatologischen Zubereitungen**
Use of cross-linked cationic polymers in skin cosmetic compositions and in dermatological compositions
Utilisation de polymères réticulés dans des compositions dermo-cosmétiques et dermatologiques

(30) Priorität: 29.06.1999 DE 19929758
(43) Veröffentlichungstag der Anmeldung: 03.01.2001
(73) Patentinhaber: BASF Aktiengesellschaft, 67056 Ludwigshafen (DE)
(72) Erfinder: Hössel, Peter, 67105 Schifferstadt (DE); Tiefensee, Kristin, 67368 Westheim (DE); Sanner, Axel, 67227 Frankenthal (DE); Dienig, Reinhold, 67105 Schifferstadt (DE); Gotsche, Michael, 52066 Aachen (DE); Zeitz, Katrin, 67067 Ludwigshafen (DE)
(74) Vertreter: Kinzebach, Werner

(56) Entgegenhaltungen:
- EP-A- 0 687 694
- EP-A- 0 893 117
- EP-A- 0 913 143
- WO-A-93/22358
- DE-A- 19 626 657
- US-A- 5 094 867

## Beschreibung

Die vorliegende Erfindung betrifft die Verwendung von vernetzten kationischen Copolymeren in Hautpflegemitteln, sowie Hautpflegemittel, welche wenigstens eines dieser Copolymere enthalten.

Kationische Polymere werden häufig als Konditioniermittel in haarkosmetischen Formulierungen eingesetzt. Sie bewirken in erster Linie eine Verbesserung der Naßkämmbarkeit des Haares. Außerdem verhindern kationische Polymere die elektrostatische Aufladung des Haares.

So wird z.B. in der EP-A-0 246 580 die Verwendung von unvernetzten Homo- und Copolymeren von 3-Methyl-1-vinylimidazoliumchloriden in kosmetischen Mitteln beschrieben. Die EP-A-0 544 158 und US-A-4,859,756 beanspruchen die Verwendung von unvernetzten Homo- und Copolymeren von chloridfreien, quaternisierten N-Vinylimidazolen in kosmetischen Zubereitungen. Aus der EP-A-0 715 843 ist die Verwendung von unvernetzten Copolymeren aus einem quaternisierten N-Vinylimidazol, N-Vinylcaprolactam und N-Vinylpyrrolidon sowie optional einem weiteren Comonomer in kosmetischen Zubereitungen bekannt.

Die DE-A-28 21 239 (US-A-4,348,380) beschreibt Copolymere von quaternisierten Diallylammoniumverbindungen in haarkosmetischen Zubereitungen. Die Polymere sind unvernetzt.

Die DE-A-31 06 974 beschreibt ein Haarbehandlungsmittel vom Vorshampooniertyp, das unvernetzte Homo- und Copolymere quaternisierter Diallylammoniumverbindungen enthält.

Die US-A-5,275,809, EP-A-0 522 755, EP-A-0 521 665 und EP-A-0 521 666 offenbaren Copolymere mit Dimethyldiallylammoniumchlorid für die Verwendung in Shampoos. In keiner der vorstehend genannten Schriften ist ein vernetztes Polymer beschrieben.

Die US-A-4,806,345 beschreibt vernetzte kationische Verdicker für kosmetische Formulierungen aus quaterniertem Dimethylaminoethylmethacrylat und Acrylamid.

Die WO 93/25595 beschreibt vernetzte kationische Copolymere auf Basis quaternisierter Dialkylaminoalkylacrylate oder Dialkylaminoalkylacrylamiden. Als Anwendung wird der Einsatz dieser vernetzten Copolymere als Verdicker in kosmetischen Zubereitungen vorgeschlagen.

Ein Verfahren zur Herstellung wasserlöslicher oder wasserquellbarer Polymerer in einer W/O-Emulsion beansprucht die EP-A-0 126 528, das dadurch gekennzeichnet ist, daß die wasserlöslichen Monomere in Anwesenheit von Emulgatoren unter Zusatz eines speziellen Dispergiersystems, bestehend aus Alkanolen, polymerisiert werden. Es werden u.a. auch kationische Comonomere eingesetzt. Als Ölphase dienen aliphatische und aromatische Kohlenwasserstoffe bzw. höhere aliphatische Ester. Für Kosmetikanwendungen sind die Polymerisate nicht vorgesehen.

In der DE-A-197 49 618 werden anionische Copolymere beschrieben, die in kosmetischen Ölen in umgekehrter Suspensionspolymerisation hergestellt, und direkt in kosmetische Formulierungen eingesetzt werden.

Die DE-A-32 09 224 beschreibt die Herstellung von vernetzten Polymerisaten auf Basis N-Vinylpyrrolidon und (quaternisiertem) N-Vinylimidazol. Diese Polymerisate werden für die Verwendung als Adsorbentien und Ionenaustauscher beansprucht.

Vernetzte, agglomerierte Vinylimidazol-Copolymerisate werden in der WO 96/26229 als Farbstoffübertragungsinhibitoren genannt. Sie sind hochvernetzt, wasserunlöslich, wenig quellbar und daher nicht geeignet als Konditioniermittel oder Gelbildner in kosmetischen Formulierungen.

Aus der US-A-4,058,491 sind vernetzte kationische Hydrogele aus N-Vinylimidazol oder N-Vinylpyrrolidon und einem quaternisierten basischen Acrylat sowie weiteren Comonomeren bekannt. Diese Gele werden zur Komplexierung und kontrollierten Freisetzung anionischer Wirksubstanzen vorgeschlagen.

Die WO 96/37525 beschreibt die Herstellung von vernetzten Copolymeren aus u.a. N-Vinylpyrrolidon und quaternisierten Vinylimidazolen in Gegenwart von Polymerisationsreglern und ihre Verwendung insbesondere in Waschmitteln. Als Gelbildner sind die Verbindungen ungeeignet.

Die DE-A-42 13 971 beschreibt Copolymerisate aus einer ungesättigten Carbonsäure, quaternisiertem Vinylimidazol und optional weiteren Monomeren und einem Vernetzer. Die Polymere werden als Verdickungs- und Dispergiermittel vorgeschlagen.

Die Verfahrensweise des Verdickens durch Protonierung eines wasserlöslichen, vernetzten Aminoalkyl(meth)acrylat wird in der EP-A-0 624 617 und EP-A-0 027 850 beschrieben.

Die Anwendung von Copolymeren mit einem Aminoalkyl(meth)acrylat in der Kosmetik ist in der EP-A-0 671 157 beschrieben. Die dort erwähnten Polymere werden aber ausschließlich für die gemeinsame Anwendung mit Festiger- oder Conditionerpolymeren verwendet.

Die WO 97/35544 beschreibt die Verwendung von vernetzten kationischen Polymeren mit Dialkylaminoalkyl(meth)acrylaten bzw. -(meth)acrylamiden in Shampoozusammensetzungen.

Die EP-A-0 893 117 nennt die Verwendung von hochmolekularen kationischen Polymeren, die bi- oder mehrfunktionelle Monomere enthalten, als Konditioniermittel in haarkosmetischen Zubereitungen.

Die DE-A-197 31 907 beschreibt die Verwendung von vernetzten kationischen Copolymeren, die N-Vinylimidazole enthalten, in haarkosmetischen Formulierungen.

Aufgabe der vorliegenden Erfindung war es, neuartige kosmetische Mittel bereitzustellen, welche eine verbesserte Hautpflege ermöglichen.

Überraschenderweise konnte diese Aufgabe gelöst werden durch Verwendung der aus der DE-A-19 731 764 bzw. der EP-A-0 893 117 und DE-A-197 31 907 bekannten und dort für die Haarbehandlung vorgeschlagenen vernetzten kationischen Copolymere.

Ein erster Gegenstand der Erfindung betrifft die Verwendung von vernetzten Copolymeren, die erhältlich sind durch (i) radikalisch initiierte Copolymerisation eines Monomergemisches aus
(a) 1 bis 99,99 Gew.-%, vorzugsweise 2 bis 94,98 Gew.-%, besonders bevorzugt 10 bis 70 Gew.-% wenigstens eines Monomers ausgewählt unter N-Vinylimidazolen und Diallylaminen, gegebenenfalls in partiell oder vollständig quaternisierter Form;
(b) 0 bis 98,99 Gew.-%, vorzugsweise 5 bis 97,98 Gew.-%, besonders bevorzugt 20 bis 89,95 Gew.-% wenigstens eines von (a) verschiedenen neutralen oder basischen wasserlöslichen Monomers,
(c) 0 bis 50 Gew.-%, vorzugsweise 0 bis 40 Gew.-%, besonders bevorzugt 0 bis 30 Gew.-% wenigstens einer ungesättigten Säure oder eines ungesättigten Anhydrids,
(d) 0 bis 50 Gew.-%, vorzugsweise 0 bis 40 Gew.-%, besonders bevorzugt 0 bis 30 Gew.-% wenigstens eines weiteren von (a), (b) und (c) verschiedenen radikalisch copolymerisierbaren Monomers und
(e) 0,01 bis 10 Gew.-%, vorzugsweise 0,02 bis 8 Gew.-%, besonders bevorzugt 0,05 bis 5 Gew.-% wenigstens eines bi- oder polyfunktionellen radikalisch copolymerisierbaren Monomers,
und (ii) anschließende teilweise oder vollständige Quaternisierung oder Protonierung des erhaltenen Copolymeren, sofern als Monomer (a) ein nicht oder nur partiell quaternisiertes Monomer eingesetzt wird, als Zusatz für hautkosmetische und dermatologische Zubereitungen zur Hautpflage.

Ein weiterer Gegenstand der Erfindung betriff eine hautkosmetische oder dermatologische Zubereitung zur Hautpflage enthaltend mindestens einen Fettkörper sowie neben üblichen Zusätzen ein kationisches copolymer, wobei das monomer (a) unter N-Vinylimidazolen ausgewählt ist.

Geeignete Monomere (a) sind die N-Vinylimidazol-Derivate der allgemeinen Formel (I), 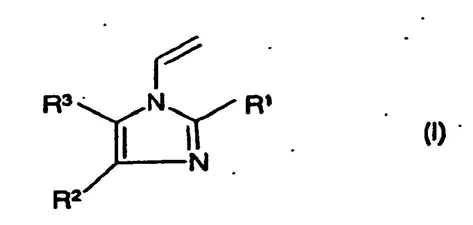 worin die Reste R¹ bis R³ unabhängig voneinander für Wasserstoff, C₁-C₄-Alkyl oder Phenyl stehen.

Weiterhin eignen sich Diallylamine der allgemeinen Formel (II), 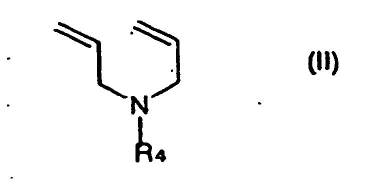 worin R⁴ für C₁-C₂₄-Alkyl steht.

Die erfindungsgemäßen Copolymere weisen gegenüber den gemäß Stand der Technik verwendeten Copolymeren auf Basis von N,N-Dialkylaminoalkyl(meth)acrylaten bzw. -(meth)acrylamiden den Vorteil auf, daß sie in wässeriger Lösung nicht hydrolysieren und damit besonders stabil sind.

Beispiele für Verbindungen der allgemeinen Formel (I) sind folgender Tabelle 1 zu entnehmen:

**Tabelle 1**

| R¹ | R² | R³ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl | | |
| Ph = Phenyl | | |

Weitere brauchbare Monomere der Formel (I) sind die Ethyl-, Propyl- oder Butyl-Analoga der in Tabelle 1 aufgelisteten Methylsubstituierten 1-Vinylimidazole.

Beispiele für Verbindungen der allgemeinen Formel (II) sind Diallylamine, worin R⁴ für Methyl, Ethyl, iso- oder n-Propyl, iso-, n- oder tert.-Butyl, Pentyl, Hexyl, Heptyl, Octyl, Nonyl oder Decyl steht. Beispiele für längerkettige Reste R⁴ sind Undecyl, Dodecyl, Tridecyl, Pentadecyl, Octadecyl und Icosyl.

Die Monomere (a) können entweder in quaternisierter Form als Mo-nomere eingesetzt werden oder nicht-quaternisiert polymerisiert werden, wobei man im letzteren Fall das erhaltene Copolymer entweder quaternisiert oder protoniert.

Zur Quaternisierung der Verbindungen der allgemeinen Formeln (I) und (II) eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternisierung der basischen Monomere der allgemeinen Formeln (I) und (II) kann auch mit Alkylenoxiden wie Ethylenoxid oder Propylenoxid in Gegenwart von Säuren durchgeführt werden.

Die Quaternisierung des Monomeren oder eines Polymeren mit einem der genannten Quaternisierungsmittel kann nach allgemein bekannten Methoden erfolgen.

Die Quaternisierung des Copolymeren kann vollständig oder auch nur teilweise erfolgen. Der Anteil quaternisierter Monomere (a) im Copolymeren kann über einen weiten Bereich variieren und liegt z.B. bei etwa 20 bis 100 Mol.-%.

Bevorzugte Quaternierungsmittel sind Methylchlorid, Dimethylsulfat oder Diethylsulfat.

Bevorzugte Beispiele für Monomere (a) sind 3-Methyl-1-vinylimidazoliumchlorid und -methosulfat, Dimethyldiallylammoniumchlorid.

Besonders bevorzugte Monomere (a) sind 3-Methyl-1-vinylimidazoliumchlorid und -methosulfat.

Zur Protonierung eignen sich beispielsweise Mineralsäuren wie HCl, H₂SO₄, H₃PO₄, sowie Monocarbonsäuren, wie z.B. Ameisensäure und Essigsäure, Dicarbonsäuren und mehrfunktionelle Carbonsäuren, wie z.B. Oxalsäure und Zitronensäure, sowie alle anderen protonenabgebenden Verbindungen und Substanzen, die in der Lage sind, das entsprechende vinylimidazol oder Diallylamin zu protonieren. Insbesondere eignen sich wasserlösliche Säuren zur Protonierung.

Die Protonierung des Polymers kann entweder im Anschluß an die Polymerisation erfolgen oder bei der Formulierung der kosmetischen Zusammenstellung, bei der in der Regel ein physiologisch verträglicher pH-Wert eingestellt wird.

Unter Protonierung ist zu verstehen, daß mindestens ein Teil der protonierbaren Gruppen des Polymers, bevorzugt 20 bis 100 Mol-%, protoniert wird, so daß eine kationische Gesamtladung des Polymers resultiert.

Geeignete von (a) verschiedene Monomere (b) sind N-Vinyllactame, wie z.B. N-Vinylpiperidon, N-Vinylpyrrolidon und N-Vinylcaprolactam, N-Vinylacetamid, N-Methyl-N-vinylacetamid, Acrylamid, Methacrylamid, N,N-Dimethylacrylamid, N-Methylolmethacrylamid, N-Vinyloxazolidon, N-Vinyltriazol, Hydroxyalkyl(meth)acrylate, wie z.B. Hydroxyethyl(meth)acrylat und Hydroxypropyl (meth) acrylate, oder Alkylethylenglykol(meth)acrylate mit 1 bis 50 Ethylenglykoleinheiten im Molekül. Außerdem eignen sich Dialkylaminoalkyl(meth)acrylate und Dialkylaminoalkyl(meth)acrylamide, wie z.B. N,N'-Dimethylaminoethylmethacrylat oder N-[3-(Dimethylamino)propyl]methacrylamid.

Bevorzugt werden als Monomere (b) N-Vinyllactame eingesetzt. Ganz besonders bevorzugt ist N-Vinylpyrrolidon.

Als Monomere (c) eignen sich ungesättigte Carbonsäuren und ungesättigte Anhydride, wie z.B. Acrylsäure, Methacrylsäure, Crotonsäure, Itaconsäure, Maleinsäure, Fumarsäure oder ihre entsprechenden Anhydride, ungesättigte Sulfonsäuren, beispielsweise Acrylamidomethylpropansulfonsäure, sowie die Salze der ungesättigten Säuren, wie z.B. die Alkali- oder Ammoniumsalze.

Als Monomere (d) eignen sich C₁-C₄₀-Alkylester der (Meth)acrylsäure, wobei die Ester abgeleitet werden von linearen, verzweigtkettigen oder carbocyclischen Alkoholen, z.B. Methyl(meth)acrylat, Ethyl(meth)acrylat, tert.-Butyl(meth)acrylat, Isobutyl(meth)acrylat, n-Butyl(meth)acrylat, Stearyl(meth)acrylat, oder Ester von alkoxylierten Fettalkoholen, z.B. C₁-C₄₀-Fettalkoholen, umgesetzt mit Ethylenoxid, Propylenoxid oder Butylenoxid, insbesondere C₁₀-C₁₈-Fettalkohole, umgesetzt mit 3 bis 150 Ethylenoxideinheiten. Weiterhin eignen sich N-Alkyl-substituierte Acrylamide mit linearen, verzweigtkettigen oder carbocyclischen Alkylresten wie N-tert.-Butylacrylamid, N-Butylacrylamid, N-Octylacrylamid, N-tert.-Octylacrylamid.

Ferner eignen sich Styrol, Vinyl- und Allylester von C₁-C₄₀-Carbonsäuren, die linear, verzweigtkettig oder carbocyclisch sein können, z.B. Vinylacetat, Vinylpropionat, Vinylneononanoat, Vinylneoundekansäure, t-Butyl-benzoesäurevinylester, Alkylvinylether, beispielsweise Methylvinylether, Ethylvinylether, Butylvinylether, Stearylvinylether;

Acrylamide, wie N-tert.-Butylacrylamid, N-Butylacrylamid, N-Octylacrylamid, N-tert.-Octylacrylamid und N-Alkyl-substituierte Acrylamide mit linearen, verzweigtkettigen oder carbocyclischen Alkylresten, wobei der Alkylrest die oben für R⁴ angegebenen Bedeutungen besitzen kann.

Monomere (e), die eine vernetzende Funktion besitzen, sind Verbindungen mit mindestens 2 ethylenisch ungesättigten, nichtkonjugierten Doppelbindungen im Molekül.

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole, wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere (e) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20 000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignete Vernetzter sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Weitere geeignete Vernetzer sind Divinyldioxan, Tetraallylsilan oder Tetravinylsilan.

Vorzugsweise werden solche Vernetzer eingesetzt, die in der Monomermischung löslich sind.

Besonders bevorzugt eingesetzte Vernetzer sind beispielsweise Pentaerythrittriallylether, Methylenbisacrylamid, Triallylamin und Triallylalkylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Methylenbisacrylamid, N,N'-Divinylethylenharnstoff, Triallylamin und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Die Monomere (a) bis (e) können jeweils einzeln oder im Gemisch mit anderen Monomeren der gleichen Gruppe eingesetzt werden.

Die Herstellung der Polymerisate erfolgt nach den an sich bekannten Verfahren der radikalisch initiierten Polymerisation, z.B. durch Lösungspolymerisation, Emulsionspolymerisation, Suspensionspolymerisation, Fällungspolymerisation, Umgekehrte Suspensionspolymerisation, Umgekehrte Emulsionspolymerisation oder durch Polymerisation in überkritischen Medien, z.B. überkritischem Kohlendioxid, ohne darauf beschränkt zu sein.

Die Polymerisation erfolgt üblicherweise bei Temperaturen von 20°C bis 150°C und bei Normaldruck oder unter Eigendruck; die Temperatur kann konstant gehalten oder kontinuierlich oder diskontinuierlich erhöht werden, z.B. um den Umsatz zu steigern.

Als Initiatoren für die radikalische Polymerisation können die hierfür üblichen wasserlöslichen und wasserunlöslichen Peroxo- und/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxid, tert.-Butylhydroperoxid, Azo-bis-isobutyronitril, Azo-bis-(2-amidinopropan)dihydrochlorid oder 2,2'-Azo-bis-(2-methylbutyronitril). Geeignet sind auch Initiatormischungen oder Redox-Initiator Systeme, wie z.B. Ascorbinsäure/Eisen(II)sulfat/Natriumperoxodisulfat, tert.-Butylhydroperoxid /Natriumdisulfit, tert.-Butylhydroperoxid/ Natriumhydroxymethansulfinat. Die Initiatoren können in den üblichen Mengen eingesetzt werden, beispielsweise 0,05 bis 5 Gew.-%, oder 0,05 bis 0,3 Mol-%, bezogen auf die Menge der zu polymerisierenden Monomeren.

Wenn die erfindungsgemäß verwendeten Copolymere in umgekehrter Suspensionspolymerisation in kosmetischen Ölen hergestellt werden, werden als Ölphase erfindungsgemäß Komponenten gewählt, die sich positiv auf die kosmetische Formulierung (Aussehen, Hautgefühl) auswirken. Solche Komponenten sind z.B. native Öle, wie Sonnenblumenöl, Mandelöl, Avocadoöl, Wachsester wie Jojobaöl, Fettsäureisopropylester wie Isopropylpalmitat, Isopropylmyristat, Di- und Triglyceride von Fettsäuren, wie z.B. Caprylsäure / Capric-glyceride. Der Anteil der Ölphase an der Gesamtemulsion beträgt 15 bis 70 Gew.-%, vorzugsweise 20 bis 35 Gew.-%.

Um die Wasserphase in der organischen Phase zu dispergieren, werden dafür bekannte W/O-Emulgatoren eingesetzt. Der HLB-Wert der verwendeten Emulgatoren liegt zwischen 4 und 8 [HLB-Wert = Hydrophilic / lipophilic balance, vgl. W.C. Giffin, J. Soc. Cosmet. Chem. 1, (1950) 311]. Solche Emulgatoren sind z.B. Sorbitanmonooleat, Sorbitanmonostearat, Glycerylmonostearat, Blockcopolymere aus Hydroxyfettsäuren-Polyestern und Polyoxyethylen. Sie können alleine oder in Kombination in Gesamtkonzentrationen von 2 bis 10 Gew.-%, vorzugsweise von 2 bis 5 Gew.-%, bezüglich der Gesamtemulsion, eingesetzt werden.

Es können der Emulsion auch Emulgatoren mit einem HLB-Wert von über 8 zugesetzt werden, und zwar in Konzentrationen von 0,25 bis 7 Gew.-% bezüglich der Gesamtemulsion. Solche Emulgatoren sind z.B. ethoxylierte C₆-C₁₂-Nonylphenole bzw. C₁₂-C₁₈-Fettalkohole; der Ethoxylierungsgrad beträgt 5 bis 20 Mol-%.

Für das Emulgieren der wässrigen Phase in die Ölphase benötigt man keine speziellen Aggregate, sondern man kann die wäßrige Monomerphase in einem Standardpolymerisationsgefäß durch Rühren, z.B. mit einem Ankerrührer emulgieren. Die Drehzahl liegt in Abhängigkeit von der Kesselgeometrie zwischen 30 und 400 Upm.

Man erhält nach der Polymerisation Wasser in Öl-Emulsionen mit einem Feststoffgehalt von 10 bis 40 Gew.-%, vorzugsweise von 15 bis 35 Gew.-%. Zur Erhöhung des Feststoffgehaltes können die Emulsionen durch Destillation teilweise oder vollständig entwässert werden.

Die erfindungsgemäß hergestellten W/O-Emulsionen vernetzter Polymerisate werden als Verdickungsmittel vorzugsweise in hautkosmetischen oder dermatologischen Anwendungen eingesetzt. Die Polymerisate werden nicht isoliert, sondern in Form der W/O-Emulsion direkt eingesetzt. Die Verdickungswirkung der W/O-Emulsion tritt direkt nach dem Vermischen der W/O-Emulsion mit einer kosmetischen O/W-Emulsion ein; um die optimale Wirkung zu erzielen, ist kein Zusatz eines Invertierungsmittels notwendig. Auch rein wäßrige Systeme lassen sich verdicken. Man erhält ein Cremegel.

Das Molekulargewicht und der K-Wert der erfindungsgemäß verwendeten Copolymerisate läßt sich in an sich bekannter Weise durch die Wahl der Polymerisationsbedingungen, beispielsweise Polymerisationsdauer, Polymerisationstemperatur oder Initiatorkonzentration, und durch den Gehalt an Vernetzer in einem breiten Bereich variieren. Die K-Werte bevorzugter Copolymerisate liegen in einem Bereich zwischen 30 bis 350, vorzugsweise 50 bis 350.

Die K-Werte werden nach Fikentscher, Cellulosechemie, Bd. 13, S. 58-64 (1932) bei 25°C 0,1%ig in 0,5 molarer Kochsalzlösung gemessen.

Bei hohen Vernetzungsgraden sind die K-Werte der Polymere nicht bestimmbar.

Die erfindungsgemäßen Polymere können in hautkosmetischen und dermatologischen nicht therapeutischen Zubereitungen eingesetzt werden.

Beispielsweise werden die erfindungsgemäßen Polymere in kosmetischen Mitteln zur Reinigung der Haut verwendet. Solche kosmetischen Reinigungsmittel sind ausgewählt aus Stückseifen, wie Toilettenseifen, Kernseifen, Transparentseifen, Luxusseifen, Deoseifen, Cremeseifen, Babyseifen, Hautschutzseifen, Abrasiveseifen und Syndets, flüssigen Seifen, wie pastöse Seifen, Schmierseifen und Waschpasten, und flüssigen Wasch-, Dusch-, und Badepräparaten, wie Waschlotionen, Duschbädern und -gelen, Schaumbädern, Ölbädern und Scrub-Präparaten.

Bevorzugt werden die erfindungsgemäßen Polymere in kosmetischen Mitteln zur Pflege und zum Schutz der Haut, in Nagelpflegemitteln sowie in Zubereitungen für die dekorative Kosmetik angewendet.

Besonders bevorzugt ist die Verwendung in Hautpflegemitteln, Intimpflegemitteln, Fußpflegemitteln, Lichtschutzmitteln, Repellents, Rasiermitteln, Haarentfernungsmitteln, Antiaknemitteln, Make-up, Maskara, Lippenstifte, Lidschatten, Kajalstiften, Eyelinern, Rouges, Pudern und Augenbrauenstiften.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

In den kosmetischen und dermatologischen Zubereitungen können die erfindungsgemäßen Polymere besondere Wirkungen enfalten. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Die erfindungsgemäßen Copolymere sind in den hautkosmetischen und dermatologischen Zubereitungen in einem Anteil von etwa 0,001 bis 20 Gew.-%, vorzugsweise 0,01 bis 10 Gew.-%, ganz besonders bevorzugt 0,1 bis 5 Gew.-%, bezogen auf das Gesamtgewicht des Mittels, enthalten.

Je nach Anwendungsgebiet können die erfindungsgemäßen Mittel in einer zur Hautpflege geeigneten Form, wie z.B. als Creme, Schaum, Gel, Stift, Pulver, Mousse, Milch oder Lotion appliziert werden.

Die hautkosmetischen Zubereitungen können neben den erfindungsgemäßen Polymeren und geeigneten Lösungsmitteln noch in der Kosmetik übliche Zusätze, wie Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel (z.B. Dihydroxyaceton), Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive enthalten.

Als geeignete Lösungsmittel sind insbesondere zu nennen Wasser und niedrige Monoalkohole oder Polyole mit 1 bis 6 Kohlenstoffatomen oder Mischungen davon; bevorzugte Monoalkohole oder Polyole sind Ethanol, i-Propanol, Propylenglycol, Glycerin und Sorbit.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀-Fettsäuren, Wachsester, wie z.B. Jojobaöl, Fettalkohole, Vaseline, hydriertes Lanolin und azetyliertes Lanolin. Selbstverständlich können auch Mischungen derselben verwendet werden.

Übliche Verdickungsmittel in derartigen Formulierungen sind vernetzte Polyacrylsäuren und deren Derivate, Polysaccharide wie Xanthan-Gum, Agar-Agar, Alginate oder Tylosen, Carboxymethylcellulose oder Hydroxycarboxymethylcellulose, Fettalkohole, Monoglyceride und Fettsäuren, Polyvinylakolhol und Polyvinylpyrrolidon.

Man kann die erfindungsgemäßen Polymere auch mit herkömmlichen Polymeren abmischen, falls spezielle Eigenschaften eingestellt werden sollen.

Als herkömmliche Polymere eignen sich beispielsweise anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer® 100P), Copolymere aus Ethylacrylat und Methacrylsäure (z.B. Luvimer® MAE), Copolymere aus N-tert.Butylacrylamid, Ethylacrylat, Acrylsäure (Ultrahold® 8, strong), Copolymere aus Vinylacetat, Crotonsäure und gegebenenfalls weitere Vinylester (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM), Copolymere von Acrylsäure und Methacrylsäure mit hydrophoben Monomeren, wie z.B. C₄-C₃₀-Alkylester der (Meth)acrylsäure, C₄-C₃₀-Alkylvinylester, C₄-C₃₀-Alkylvinylether und Hyaluronsäure.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7) und Chitosan.

Als weitere Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die erfindungsgemäßen Copolymerisate werden in kosmetischen oder dermatologischen Zubereitungen eingesetzt, deren Herstellung nach den üblichen dem Fachmann geläufigen Regeln erfolgt.

Solche Formulierungen liegen vorteilhafterweise in Form von Emulsionen bevorzugt als Wasser-in-Öl-(W/O)- oder Öl-in-Wasser(O/W)-Emulsionen vor. Es ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung erfindungsgemäß brauchbarer Emulsionen erfolgt nach bekannten Methoden.

Die Emulsionen enthalten neben dem erfindungsgemäßen Copolymer übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil, worauf hiermit ausdrücklich Bezug genommen wird.

So kann eine erfindungsgemäß brauchbare Hautcreme z.B. als W/O-Emulsion vorliegen. Eine derartige Emulsion enthält eine wäßrige Phase, die mittels eines geeigneten Emulgatorsystems in einer Öl- oder Fettphase emulgiert ist.

Die Konzentration des Emulgatorsystems beträgt in diesem Emulsions-Typ etwa 4 bis 35 Gew.-%, bezogen auf das Gesamtgewicht der Emulsion; die Fettphase macht etwa 20 bis 60 Gew. -% aus und die wäßrige Phasen etwa 20 bis 70 Gew.-%, jeweils bezogen auf das Gesamtgewicht der Emulsion. Bei den Emulgatoren handelt es sich um diejenigen, welche in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glyzerin oder Polyglyzerin; Kondensaten von Ethylenoxid und Propylenglycolen; oxypropylenierten/oxyethylenierten C₁₂-C₂₀-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink oder Aluminiumlanolat und hydriertem Lanolin oder Lanolinalkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwachs und Ca-, Mg- und Al-Oleate, -Myristate, -Linoleate und -Stearate.

Im allgemeinen werden diese Wasser-in-Öl-Emulsionen so hergestellt, daß die Fettphase und der Emulgator in den Ansatzbehälter gegeben werden. Man erwärmt diesen bei einer Temperatur von 70 bis 75°C, gibt dann die in Öl löslichen Ingredienzien zu und fügt unter Rühren Wasser hinzu, welches vorher auf die gleiche Temperatur erwärmt wurde und worin man die wasserlöslichen Ingredienzien vorher gelöst hat; man rührt, bis man eine Emulsion der gewünschten Feinheit hat, läßt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wäßrige Phase, die üblicherweise verdickt vorliegt.

Die wäßrige Phase der O/W-Emulsion der erfindungsgemäßen Zubereitungen enthält gegebenenfalls
- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z.B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:
- Ester aus gesättigten und/oder ungesättigten, verzweigten und/oder unverzweigten C₃-C₃₀-Alkancarbonsäuren und gesättigten und/oder ungesättigten, verzweigten und/oder unverzweigten C₃-C₃₀-Alkoholen, aus aromatischen Carbonsäuren und gesättigten und/oder ungesättigten, verzweigten und/oder unverzweigten C₃-C₃₀-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter C₈-C₂₄-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

Die erfindungsgemäßen Copolymerisate eignen sich auch zur Verwendung in Wasch- und Duschgel-Formulierungen sowie Badepräparaten.

Solche Formulierungen enthalten neben den erfindungsgemäßen Polymeren üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

In den Wasch, Dusch- und Badepräparaten können alle in Körperreinigungsmitteln üblicherweise eingesetzte anionische, neutrale, amphotere oder kationische Tenside verwendet werden.

Die Formulierungen enthalten 2 bis 50 Gew.-% Tenside, bevorzugt 5 bis 40 Gew.-%, besonders bevorzugt 8 bis 30 Gew-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside oder Sorbitanetherester geeignet.

Außerdem können die Wasch, Dusch- und Badepräparate übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), Guar-hydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaternium-16, -44, -46), Copolymere aus N-Vinypyrrolidon/Dimethylaminoethyl-methacrylat, quaternisiert mit Diethylsulfat (Polyquaternium-11) und andere.

Weiterhin können die Wasch- und Duschgel-Formulierungen und Badepräparate Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

### A Herstellung der Polymere

### Herstellungsbeispiel 1

In einer Rührapparatur wurden 400 g Wasser und 46 g Dimethyldiallylammoniumchlorid -Lösung (65%ig) vorgelegt. Zu dieser Vorlage wurde 10% von Zulauf 1, bestehend aus 270 g N-Vinylpyrrolidon und 0,6 g N,N'-Divinylethylenharnstoff, gegeben. Unter Rühren im Stickstoffstrom wurde auf 60°C aufgeheizt und Zulauf 1 während 3 Stunden sowie Zulauf 2, bestehend aus 0,9 g 2,2'-Azo-bis(2-amidinopropan)dihydrochlorid in 100 g Wasser, während 4 Stunden zudosiert. Nach 3 Stunden verdünnte man mit 700 g Wasser und ließ eine weitere Stunde rühren. Danach gab man 1,5 g 2,2'-Azo-bis(2-amidinopropan)dihydrochlorid in 30 g Wasser zu und ließ weitere 2 Stunden bei 60°C rühren. Man erhielt eine farblose hochviskose Polymerlösung mit einem Feststoffgehalt von 20,9% und einem K-Wert von 80,3.

### Herstellungsbeispiel 2

In einer Rührapparatur wurden 300 g von Zulauf 1, bestehend aus 200 g N-Vinylpyrrolidon, 77 g Dimethyldiallylammoniumchlorid-Lösung (65%ig), 1,13 g N,N'-Divinylethylenharnstoff und 440 g Wasser, vorgelegt und unter Rühren im Stickstoffstrom auf 60°C aufgeheizt. Der Rest von Zulauf 1 wurde in 2 Stunden und Zulauf 2, bestehend aus 0,75 g 2,2'-Azo-bis(2-amidinopropan)dihydrochlorid in 100 g Wasser, in 4 Stunden zudosiert. Nach Ende von Zulauf 1 wurde das Reaktionsgemisch mit 1620 g Wasser verdünnt. Nach Ende von Zulauf 2 ließ man eine weitere Stunde bei 60°C rühren, gab danach 1,25 g 2,2'-Azo-bis(2-amidinopropan)dihydrochlorid in 65 g Wasser zu und rührte eine weitere Stunde. Man erhielt eine farblose hochviskose Polymerlösung mit einem Feststoffgehalt von 10,2% und einem K-Wert von 80.

### Herstellungsbeispiel 3

In einer Rührapparatur wurden 130 g Wasser und 48 g 3-Methyl-1-vinylimidazoliumchlorid vorgelegt und unter Rühren im Stickstoffstrom auf 60°C aufgeheizt. Danach wurden Zulauf 1, bestehend aus 192 g N-Vinylpyrrolidon, 0,48 g N,N'-Divinylethylenharnstoff und 450 g Wasser, in 3 Stunden und Zulauf 2, bestehend aus 1,44 g 2,2'-Azo-bis(2-amidinopropan)dihydrochlorid in 80 g Wasser, während 4 Stunden zudosiert. Anschließend wurde noch eine Stunde bei 60°C gerührt. Um den Ansatz rührfähig zu halten, wurde nach Bedarf mit insgesamt 2100 g Wasser verdünnt. Man erhielt eine farblose hochviskose Polymerlösung mit einem Feststoffgehalt von 8,2% und einem K-Wert von 105.

### Herstellungsbeispiel 4

In einer Rührapparatur wurden 716 g Wasser vorgelegt und unter Rühren im Stickstoffstrom auf 60°C aufgeheizt. Danach wurde Zulauf 1, bestehend aus 180 g N-Vinylpyrrolidon, 20 g 3-Methyl-1-vinylimidazoliummethylsulfat, 0,32 g N,N'-Divinylethylenharnstoff und 25 g Wasser, in 2 Stunden und Zulauf 2, bestehend aus 0,6 g 2,2'-Azo-bis(2-amidinopropan)dihydrochlorid in 60 g Wasser, in 3 Stunden zudosiert. Nach Ende von Zulauf 1 wurde das Reaktionsgemisch mit 1000 g Wasser verdünnt. Im Anschluß an Zulauf 2 wurde noch 3 Stunden bei 70°C gerührt. Man erhielt eine farblose hochviskose Polymerlösung mit einem Feststoffgehalt von 11,0% und einem K-Wert von 86.

### Herstellungsbeispiel 5

In einer Rührapparatur wurden 440 g Wasser vorgelegt und unter Rühren im Stickstoffstrom auf 60°C aufgeheizt. Danach wurde Zulauf 1, bestehend aus 180 g N-Vinylpyrrolidon, 20 g 3-Methyl-1-vinylimidazoliummethylsulfat, 0,30 g N,N'-Divinylethylenharnstoff und 25 g Wasser, in 2 Stunden und Zulauf 2, bestehend aus 0,6 g 2,2'-Azo-bis(2-amidinopropan)dihydrochlorid in 60 g Wasser, in 3 Stunden zudosiert. Im Anschluß an Zulauf 2 wurde noch 3 Stunden bei 70°C gerührt. Um das Reaktionsgemisch rührfähig zu halten, wurde bei Bedarf insgesamt mit 1275 g Wasser verdünnt. Man erhielt eine farblose hochviskose Polymerlösung mit einem Feststoffgehalt von 11,3% und einem K-Wert von 105.

### Herstellungsbeispiel 6

In einer Rührapparatur wurden 650 g Wasser vorgelegt und unter Rühren im Stickstoffstrom auf 60°C aufgeheizt. Danach wurde Zulauf 1, bestehend aus 225 g N-Vinylpyrrolidon, 25 g 2,3-Dimethyl-1-vinylimidazoliummethylsulfat, 0,25 g N,N'-Divinylethylenharnstoff und 580 g Wasser, in 3 Stunden und Zulauf 2, bestehend aus 0,7 g 2,2'-Azo-bis(2-amidinopropan)dihydrochlorid in 100 g Wasser, in 4 Stunden zudosiert. Nach Ende von Zulauf 1 wurde das Reaktionsgemisch mit 835 g Wasser verdünnt. Im Anschluß an Zulauf 2 wurde noch eine Stunde gerührt und 1,25 g 2,2'-Azo-bis(2-amidinopropan)dihydrochlorid in 77 g Wasser nachdosiert. Danach rührte man noch 2 Stunden bei 70°C. Man erhielt eine farblose hochviskose Polymerlösung mit einem Feststoffgehalt von 10,4% und einem K-Wert von 106.

### Herstellungsbeispiel 7

In einer Rührapparatur wurden 650 g Wasser vorgelegt und unter Rühren im Stickstoffstrom auf 60°C aufgeheizt. Danach wurde Zulauf 1, bestehend aus 225 g N-Vinylpyrrolidon, 25 g 2,3-Dimethyl-1-vinylimidazoliummethylsulfat, 0,375 g N,N' -Divinylethylenharnstoff und 580 g Wasser, in 3 Stunden und Zulauf 2, bestehend aus 0,7 g 2,2'-Azo-bis(2-amidinopropan)dihydrochlorid in 100 g Wasser, während 4 Stunden zudosiert. Nach Ende von Zulauf 1 wurde das Reaktionsgemisch mit 1135 g Wasser verdünnt. Im Anschluß an Zulauf 2 wurde noch eine Stunde gerührt und 1,25 g 2,2'-Azo-bis(2-amidinopropan)dihydrochlorid in 77 g Wasser nachdosiert. Danach rührte man noch 2 Stunden bei 70°C. Man erhielt eine farblose hochviskose Polymerlösung mit einem Feststoffgehalt von 9,2% und einem K-Wert von 92.

### Herstellungsbeispiel 8

In einem Reaktionsgefäß mit Stickstoffspülung wurden 800 g Cyclohexan, 5 g Sorbitanmonooleat, 5 g Hypermer B246 (Hypermer B246: polymeres Tensid der Fa. ICI) und 1 g 2,2'-Azobis(2,4-dimethylvaleronitril) vorgelegt und auf 65°C erwärmt. Der Zulauf, bestehend aus 100 g 3-Methyl-1-vinylimidazoliummethylsulfat, 100 g N-Vinylpyrrolidon, 100 g Wasser und 0,25 g Tripropylenglycoldiacrylat, wurde innerhalb 20 Minuten zudosiert. Anschließend wurde 6 Stunden bei 65°C gerührt. Dann wurden 200 g Cyclohexan zugesetzt und das Wasser azeotrop abdestilliert, das Polymerisat abfiltriert und getrocknet. Der K-Wert einer wässerigen Lösung des Polymerisates betrug 114.

### Herstellungsbeispiel 9

In einer Rührapparatur wurden 900 g Ethylacetat vorgelegt und unter Rühren im Stickstoffstrom auf 77°C aufgeheizt. Danach wurde Zulauf 1, bestehend aus 270 g N-Vinylpyrrolidon, 30 g 1-Vinylimidazol und 0,3 g N,N'-Divinylethylenharnstoff, in 3 Stunden und Zulauf 2, bestehend aus 3 g 2,2'-Azo-bis(2-methylbutyronitril) in 80 g Ethylacetat, während 4 Stunden zudosiert. Danach rührte man noch 2 Stunden, kühlte auf Raumtemperatur ab und versetzte mit 36 g Dimethylsulfat. Anschließend rührte man eine halbe Stunde bei Raumtemperatur und weitere 2 Stunden bei 70°C. Das erhaltene Pulver wurde abfiltriert und getrocknet. Der K-Wert einer wässerigen Lösung des Polymerisates betrug 125.

### Herstellungsbeispiel 10

In einer Rührapparatur wurden 440 g Wasser vorgelegt und unter Rühren im Stickstoffstrom auf 60°C aufgeheizt. Danach wurde Zulauf 1, bestehend aus 144 g N-Vinylpyrrolidon, 16 g 3-Methyl-1-vinylimidazoliummethylsulfat, 1,4 g Tetraethylenglykoldiacrylat und 100 g Wasser, in 2 Stunden und Zulauf 2, bestehend aus 0,8 g 2,2'-Azo-bis(2-amidinopropan)dihydrochlorid in 50 g Wasser, in 3 Stunden zudosiert. Im Anschluß an Zulauf 2 wurde noch 3 Stunden bei 70°C gerührt. Um das Reaktionsgemisch rührfähig zu halten, wurde bei Bedarf insgesamt mit 1200 g Wasser verdünnt. Man erhielt eine farblose, hochviskose Polymerlösung mit einem Feststoffgehalt von 8,5% und einem K-Wert von 95.

### Herstellungsbeispiel 11

In einer Rührapparatur wurden 550 g Wasser vorgelegt und unter Rühren im Stickstoffstrom auf 60°C aufgeheizt. Danach wurde Zulauf 1, bestehend aus 102 g N-Vinylpyrrolidon, 26 g 3-Methyl-1-vinylimidazoliummethylsulfat, 0,8 g Triallylamin und 100 g Wasser in 2 Stunden zudosiert. Zulauf 2, bestehend aus 0,6 g 2,2'-Azo-bis(2-amidinopropan)dihydrochlorid in 50 g Wasser, wurde in 3 Stunden zur Reaktionsmischung zugegeben. Im Anschluß an Zulauf 2 wurde noch 3 Stunden bei 70°C gerührt. Um das Reaktionsgemisch rührfähig zu halten, wurde bei Bedarf insgesamt mit 1000 g Wasser verdünnt. Man erhielt eine schwach gelbliche, hochviskose Polymerlösung mit einem Feststoffgehalt von 7,0% und einem K-Wert von 102.

### Herstellungsbeispiel 12

Herstellungsbeispiel 11 wurde wiederholt, nur wurde anstelle von Triallylamin 2,2 g Pentaerythrittriallylether eingesetzt. Man erhielt eine schwach gelbliche, hochviskose Polymerlösung mit einem K-Wert von 95.

### Herstellungsbeispiel 13

In einer Rührapparatur wurden 440 g Wasser vorgelegt und unter Rühren im Stickstoffstrom auf 60°C aufgeheizt. Danach wurde Zulauf 1, bestehend aus 150 g N-Vinylpyrrolidon, 8 g 3-Methyl-1-vinylimidazoliummethylsulfat, 0,6 g Triallylamin und 100 g Wasser, in 2 Stunden und Zulauf 2, bestehend aus 0,8 g 2,2'-Azo-bis(2-amidinopropan)dihydrochlorid in 50 g Wasser, in 3 Stunden zudosiert. Im Anschluß an Zulauf 2 wurde noch 3 Stunden bei 70°C gerührt. Um das Reaktionsgemisch rührfähig zu halten, wurde bei Bedarf insgesamt mit 1200 g Wasser verdünnt. Man erhielt eine farblose, hochviskose Polymerlösung mit einem Feststoffgehalt von 8,1% und einem K-Wert von 98.

### Herstellungsbeispiel 14

In einem Reaktionsgefäß mit Stickstoffspülung wurden 800 g Cyclohexan, 5 g Sorbitanmonooleat und 5 g Hypermer B246 (Hypermer B246: polymeres Tensid der Fa. ICI) vorgelegt und auf 60°C erwärmt. Zulauf 1, bestehend aus 60 g 3-Methyl-1-vinylimidazoliummethylsulfat, 140 g N-Vinylpyrrolidon, 150 g Wasser und 1,0 g Triallylamin, und Zulauf 2, bestehend aus 0,6 g 2,2'-Azo-bis(2-amidinopropan)dihydrochlorid in 50 g Wasser, wurden innerhalb 1 Stunde zudosiert. Anschließend wurde weiteren 6 Stunden bei 60°C gerührt. Dann wurden 200 g Cyclohexan zugesetzt und das Wasser azeotrop abdestilliert, das Polymerisat abfiltriert und getrocknet.

### Herstellungsbeispiel 15

In einem Reaktionsgefäß mit Stickstoffspülung wurden 800 g Cyclohexan, 5 g Sorbitanmonooleat und 5 g Hypermer B246 (Hpyermer B246: polymeres Tensid der Fa. ICI) vorgelegt und auf 60°C erwärmt. Zulauf 1, bestehend aus 20 g 3-Methyl-1-vinylimidazoliummethylsulfat, 180 g N-Vinylpyrrolidon, 150 g Wasser und 0,5 g Triallylamin, wurde innerhalb 1 Stunde und Zulauf 2, bestehend aus 1,2 g 2,2'-Azo-bis(2-amidinopropan)dihydrochlorid in 70 g Wasser, innerhalb 4 Stunden zudosiert. Anschließend wurde weitere 3 Stunden bei 60°C gerührt. Dann wurden 200 g Cyclohexan zugesetzt und das Wasser azeotrop abdestilliert, das Polymerisat abfiltriert und getrocknet.

### Herstellungsbeispiel 16

In einer Rührapparatur wurden 400 g Wasser, 100 g N-Vinylpyrrolidon, 11 g 3-Methyl-1-vinylimidazoliummethylsulfat und 0,4 g Triallylamin vorgelegt und unter Rühren im Stickstoffstrom auf 60°C aufgeheizt. Danach wurde Zulauf 1, bestehend aus 0,6 g 2,2'-Azo-bis(2-amidinopropan)dihydrochlorid in 50 g Wasser, in 3 Stunden zur Reaktionsmischung zugegeben und mit 1000 g Wasser verdünnt. Anschließend wurde noch 3 Stunden bei 80°C gerührt. Man erhielt eine farblose, hochviskose Polymerlösung mit einem Feststoffgehalt von 7,6% und einem K-Wert von 110.

### Herstellungsbeispiel 17

In einem Reaktionsgefäß wurden 500 g Cyclohexan, 12 g Sorbitanmonooleat, 6 g Hypermer B246 (Hypermer B246: polymeres Tensid der Fa. ICI) und einer wässrigen Phase aus 150 g 3-Methyl-1-vinylimidazoliummethylsulfat, 150 g Vinylpyrrolidon, 0,75 g 2,2'-Azo-bis(2-amidinopropan)-dihydrochlorid, 360 g Wasser und 0,6 g Divinylethylenharnstoff, 6 h bei 70°C polymerisiert. Danach wurde das Wasser azeotrop abdestilliert, das Polymerisat abfiltriert und getrocknet.

### Herstellungsbeispiel 18

In einem Reaktionsgefäß wurden 200 g Mygliol (Caprylic/capric-Triglyceride der Hüls AG), 12 g Sorbitanmonooleat, 6 g Hypermer B246 (ICI) und einer wässrigen Phase aus 333 g Vinylimidazolium-Methylsulfat, 150 g vinylpyrrolidon, 0,75 g 2,2'-Azobis(2-amidinopropan)-dihydrochlorid, 186 g Wasser und 0,6 g Divinylethylenharnstoff, 6 h bei 70°C polymerisiert.

### Herstellungsbeispiel 19

In einer Rührapparatur wurden 500 g Wasser vorgelegt und auf 60°C erhitzt. Danach wurde Zulauf 1, bestehend aus 125 g Vinylimidazol, 48 g Acrylsäure, 42 g NaOH (50%ige wässrige Lösung), 0,8 g Triallylamin und 500 g Wasser, und Zulauf 2, bestehend aus 1,25 g 2,2'-Azo-bis(2-amidinopropan)dihydrochlorid und 200 g Wasser, in 4 h zugegeben. Anschließend wurde die Innentemperatur auf 70°C erhöht und 1,25 g 2,2'-Azo-bis(2-amidinopropan)dihydrochlorid in 78 g Wasser innerhalb von 1 h zugegeben und anschließend noch 1 h bei 70°C gerührt. Um das Reaktionsgemisch rührfähig zu halten, wurde bei Bedarf insgesamt mit 1200 g Wasser verdünnt. Man erhielt eine schwach gelbliche Polymerlösung mit einem Feststoffgehalt von 7,8 % und einem K-Wert von 110.

### Herstellungsbeispiel 20

Herstellungsbeispiel 19 wurde wiederholt, wobei nur anstelle von 125 g 1-Vinylimidazol 125 g 3-Methyl-1-vinylimidazoliummethylsulfat eingesetzt wurde. Man erhielt eine schwach gelbliche Polymerlösung mit einem Feststoffgehalt von 7,8 % und einem K-Wert von 105.

### B Anwendungsbeispiele

### Anwendungsbeispiel 1: Hautcreme

**Gemäß folgender Rezeptur wurde zunächst eine erfindungsgemäße Wasser/Öl-Cremeemulsion (Hautcreme A) hergestellt:**

| | Zusatz | Gew.-% |
|---|---|---|
| Cremophor A 6 | Ceteareth-6 und Stearylalkohol | 2,0 |
| Chremophor A 25 | Ceteareth-25 | 2,0 |
| Lanette O | Cetearylalkohol | 2,0 |
| Imwitor 960 K | Glycerylstearat SE | 3,0 |
| Paraffinöl | | 5,0 |
| jojobaöl | | 4,0 |
| Luvitol EHO | Cetearyloctanoat | 3,0 |
| ABIL 350 | Dimethicone | 1,0 |
| Amerchol L 101 | Mineralöl und Lanolinalkohol | 3,0 |
| Veegum Ultra | Magnesiumaluminiumsilikat | 0,5 |
| 1,2-Propylenglykol | Propylenglykol | 5,0 |
| Abiol | Imidazolindinyl-Harnstoff | 0,3 |
| Phenoxyethanol | | 0,5 |
| D-Panthenol USP | | 1,0 |
| Polymer (Herstellungsbeispiel 11) | | 0,5 |
| Wasser | | ad 100 |

In gleicher Weise wurden zwei Vergleichscremes hergestellt, und zwar:

### Hautcreme B (ohne Polymerzusatz)

### Hautcreme C (erfindungsgemäßes Polymer ersetzt durch die gleiche Menge eines unvernetzten Copolymers aus 30 Mol.-% vinylimidazoliumchlorid und 70 Mol.-% N-Vinylpyrrolidon)

Mit diesen Hautcremes A, B und C wurden die folgenden Vergleichstests 1 und 2 zur Beurteilung des Hautgefühls durchgeführt.

100 µl der Emulsion wurden gleichmäßig auf dem Handrücken verteilt und das Hautgefühl subjektiv nach 30 Minuten Einwirkzeit getestet. Es wurden jeweils zwei Emulsionen (rechte-linke Hand) miteinander verglichen. Der Test wurde von je 10 Probanden durchgeführt.

### Notenskala:

2 (deutlich zarter als Vergleichscreme)
1 (etwas zarter als Vergleichscreme)
0 (gleich)
-1 (etwas rauher als Vergleichscreme)
-2 (deutlich rauher als Vergleichscreme)

**Ergebnis von Vergleichstest 1 (Vergleich der Hautcreme A und Vergleichscreme B):**

| Note | Anzahl der Probanden |
|---|---|
| 2 | 5 |
| 1 | 4 |
| 0 | 1 |
| -1 | - |
| -2 | - |

**Ergebnis von Vergleichstest 2 (vergleich der Hautcreme A und Vergleichscreme C):**

| Note | Anzahl der Probanden |
|---|---|
| 2 | 3 |
| 1 | 5 |
| 0 | 2 |
| -1 | - |
| -2 | - |

### Anwendungsbeispiel 2: Duschgel

**Gemäß folgender Rezeptur wurde zunächst eine erfindungsgemäße Duschgel-Formulierung (Duschgel A) hergestellt:**

| | Zusatz | Gew.-% |
|---|---|---|
| Texapon NSO | Natriumlaurethsulfat | 40,0 |
| Tego Betain L7 | Cocamidopropylbetain | 5,0 |
| Plantacare 2000 | Decylglucosid | 5,0 |
| Parfüm | | 0,2 |
| Polymer gemäß Herstellungsbeispiel 11 | | 0,2 |
| Euxyl K 100 | Benzylalkohol, Methylchlorisothiazolinon, Methylisothiazolinon | 0,1 |
| D-Panthenol USP | | 0,5 |
| Citronensäure (pH 6-7) | | q.s. |
| NaCl | | 2,0 |
| Wasser | | ad 100 |

In gleicher Weise wurden drei Vergleichs-Duschgele hergestellt und zwar:
Duschgel B: (erfindungsgemäßes Copolymer ersetzt durch gleiche Menge des nicht vernetzten Polymers Polyquaternium-16)
Duschgel C: (erfindungsgemäßes Copolymer ersetzt durch gleiche Menge kationisch modifizierter Hydroxyethylcellulose)
Duschgel D: (ohne Polymerzusatz)

Mit den Duschgelen A, B, C und D wurde der folgende Vergleichstest 3 zur Bestimmung der Schaumcremigkeit durchgeführt:

Je 2,0 g der oben genannten Formulierung wurde auf die linke Handinnenfläche gegeben, mit Leitungswasser angeschäumt und nach 1 Minute Reiben zwischen beiden Händen das Schaumgefühl in den Handinnenflächen beurteilt:
Note 1: sehr cremig/sahnig
Note 2: cremig/sahnig
Note 3: stumpf/gehaltlos

**Ergebnis von Vergleichstest 3 (Mittelwert der Benotung durch 10 Probanden):**

| Duschgel | Mittelwert von 10 Probanden |
|---|---|
| A | 1,3 |
| B | 2,8 |
| C | 2,1 |
| D | 2,8 |

### Anwendungsbeispiel 3: Feuchthalteformulierung

### Formulierung A

| | Zusatz | Gew.-% |
|---|---|---|
| a) Cremophor A6 | Ceteareth-6 und Stearylalkohol | 2,0 |
| Cremophor A25 | Ceteareth-25 | 2,0 |
| Paraffinöl (dickflüssig) | | 10 |
| Lannette O | Cetearylalkohol | 2,0 |
| Stearinsäure | | 3,0 |
| Nip-Nip | Methylparaben/Propylparaben 70:30 | 0,5 |
| Abiol | Imidazolidinyl-Harnstoff | 0,5 |
| b) Polymer (Herstellungsbeispiel 8) | | 3,0 |
| Wasser | | ad 100,0 |

Beide Phasen wurden auf 80°C erhitzt, Phase a) wurde in b) eingerührt, homogenisiert und kaltgerührt und anschließend mit 10%iger wässriger NaOH-Lösung auf pH 6 eingestellt.

In gleicher Weise wurde eine Vergleichscreme (Formulierung B) ohne Polymerzusatz hergestellt.

Mit den Formulierungen A und B wurde ein Probandentest an 8 Probanden durchgeführt. Dazu wurden die Formulierungen jeweils auf den Unterarm der Probanden in einer Menge von 2 mg/cm² aufgetragen. Nach 30 min wurde der Feuchtigkeitsgehalt der Haut mit einem Corneometer CM 825 (Fa. Khazaka & Courage) bestimmt. Nach Applikation von Formulierung A wurde ein durchschnittlicher Wert von 45 Corneometereinheiten gemessen, mit Formulierung B ein durchschnittlicher Wert von 35.

### Anwendungsbeispiel 4: O/W Creme zur Hautfeuchthaltung

| Zusatz | Gew.-% |
|---|---|
| Glycerinmonostearat | 2,0 |
| Cetylalkohol | 3,0 |
| Paraffinöl, subliquidum | 15,0 |
| Vaseline | 3,0 |
| Caprylcaprinsäuretriglycerid | 4,0 |
| Octyldodecanol | 2,0 |
| Hydriertes Kokosfett | 2,0 |
| Cetylphosphat | 0,4 |
| Polymer (Herstellungsbeispiel 6) | 3,0 |
| Glycerin | 3,0 |
| Natriumhydroxid | q.s. |
| Parfümöl | q.s. |
| Konservierungsmittel | q.s. |
| Wasser | ad 100 |

### Anwendungsbeispiel 5: O/W-Lotion

| Zusatz | Gew.-% |
|---|---|
| Stearinsäure | 1,5 |
| Sorbitanmonostearat | 1,0 |
| Sorbitanmonooleat | 1,0 |
| Paraffinöl, subliquidum | 7,0 |
| cetylalkohol | 1,0 |
| Polydimethylsiloxan | 1,5 |
| Glycerin | 3,0 |
| Polymer (Herstellungsbeispiel 17) | 0,5 |
| Parfümöl | q.s. |
| Konservierungsmittel | q.s. |
| Wasser | ad 100 |

### Anwendungsbeispiel 6: W/O-Creme

| Zusatz | Gew.-% |
|---|---|
| PEG-7-hydriertes Ricinusöl | 4,0 |
| Wollwachsalkohol | 1,5 |
| Bienenwachs | 3,0 |
| Triglycerid, flüssig | 5,0 |
| Vaseline | 9,0 |
| Ozokerite | 4,0 |
| Paraffinöl, subliquidum | 4,0 |
| Glycerin | 2,0 |
| Polymer (Herstellungsbeispiel 4) | 2,0 |
| Magnesiumsulfat*7H₂O | 0,7 |
| Parfümöl | q.s. |
| Konservierungsmittel | q.s. |
| Wasser | ad 100 |

### Anwendungsbeispiel 7: Hydrogel zur Hautpflege

| Zusatz | Gew.-% |
|---|---|
| Polymer (Herstellungsbeispiel 17) | 3,0 |
| Sorbit | 2,0 |
| Glycerin | 3,0 |
| Polyethylenglycol 400 | 5,0 |
| Ethanol | 1,0 |
| Parfümöl | q.s. |
| Konservierungsmittel | q.s. |
| Wasser | ad 100 |

### Anwendungsbeispiel 8: Hydrodispersionsgel

| Zusatz | Gew.-% |
|---|---|
| Polymer (Herstellungsbeispiel 17) | 3,0 |
| Sorbit | 2,0 |
| Glycerin | 3,0 |
| Polyethylenglycol 400 | 5,0 |
| Triglycerid, flüssig | 2,0 |
| Ethanol | 1,0 |
| Parfümöl | q.s. |
| Konservierungsmittel | q.s. |
| Wasser | ad 100 |

### Anwendungsbeispiel 9: Flüssige Seife

| Zusatz | Gew.-% |
|---|---|
| Kokosfettsäure, Kaliumsalz | 15 |
| Kaliumoleat | 3 |
| Glycerin | 5 |
| Polymer (Herstellungsbeispiel 14) | 2 |
| Glycerinstearat | 1 |
| Ethylenglycoldistearat | 2 |
| Spezifische Zusätze, Komplexierungsmittel, Duftstoffe | q.s. |
| Wasser | ad 100 |

### Anwendungsbeispiel 10: Body Care Cream

| | Zusatz | Gew.-% |
|---|---|---|
| Cremophor A6 | Ceteareth-6 und Stearylalkohol | 2,0% |
| Cremophor A 25 | Ceteareth-25 | 2,0% |
| Grape (Vitis Vinifera)Seed oil | | 6,0% |
| Glycerylstearat SE | | 3,0% |
| Cetearylalkohol | | 2,0% |
| Dimethicon | | 0,5% |
| Luvitol EHO | Cetearyloctanoat | 8,0% |
| Oxynex 2004 | Propylenglycol, BHT, Ascorbylpalmitat, Glycerylstearat, Citronensäure | 0,1% |
| Konservierungsmittel | | q.s. |
| 1,2-Propylenglykol USP | | 3,0% |
| Glycerin | | 2,0% |
| EDTA BD | | 0,1% |
| D-Panthenol USP | | 1,0% |
| Wasser | | ad 100 |
| Polymer (Herstellungsbeispiel 11) | | 1,5% |
| Tocopherylacetat | | 0,5% |

Die Formulierung wies einen pH-Wert von 6,8 auf. Die Viskosität (Brookfield RVT, 23°C) betrug 32000 mPas.

## Patentansprüche

1. Nicht-therapeutische Verwendung wenigstens eines Copolymers, das erhältlich ist durch
(i) radikalisch initiierte Copolymerisation eines Monomerengemisches aus
(a) 1 bis 99,99 Gew.-% wenigstens eines Monomers, ausgewählt unter N-Vinylimidazolen und Diallylaminen, gegebenenfalls in partiell oder vollständig quaternisierter Form;
(b) 0 bis 98,99 Gew.-% wenigstens eines von (a) verschiedenen neutralen oder basischen wasserlöslichen Monomers;
(c) 0 bis 50 Gew.-% wenigstens einer ungesättigten Säure oder eines ungesättigten Anhydrids;
(d) 0 bis 50 Gew.-% wenigstens eines von (a), (b) oder (c) verschiedenen radikalisch copolymerisierbaren Monomers; und
(e) 0,01 bis 10 Gew.-% wenigstens eines als Vernetzer wirkenden Monomers mit mindestens zwei ethylenisch ungesättigten, nichtkonjugierten Doppelbindungen; und
(ii) anschließende teilweise oder vollständige Quaternisierung oder Protonierung des Polymeren für den Fall, daß das Monomer (a) nicht oder nur partiell quaternisiert ist
als Zusatz für hautkosmetische und dermatologische Zubereitungen zur Hautpflage.

2. Verwendung nach Anspruch 1, **dadurch gekennzeichnet, dass** die Menge an Monomer (c) 0 bis 40 Gew.-% beträgt.

3. Verwendung nach Anspruch 1 oder 2, **dadurch gekennzeichnet, daß** die Protonierung gemäß (ii) bei der Formulierung der Zubereitung erfolgt.

4. Verwendung nach einem der Ansprüche 1 bis 3, **dadurch gekennzeichnet daß** als Monomer (a) wenigstens ein Diallylamin-Derivat der allgemeinen Formel (II), 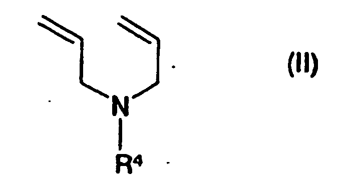 worin der Rest R⁴ für C₁ - C₂₄ -Alkyl steht, verwendet wird.

5. Verwendung nach einem der Ansprüche 1 bis 3, **dadurch gekennzeichnet daß** als Monomer (a) wenigstens ein N-Vinylimidazol-Derivat der allgemeinen Formel (I), 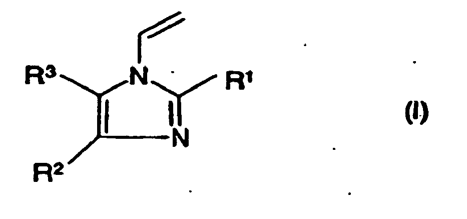 worin die Reste R¹ bis R³ unabhängig voneinander für Wasserstoff, C₁-C₄ -Alkyl oder Phenyl stehen, verwendet wird.

6. Verwendung nach einem der Ansprüche 1 bis 5, **dadurch gekennzeichnet, daß** als Monomer (b) wenigstens ein N-Vinyllactam verwendet wird.

7. Verwendung nach einem der Ansprüche 1 bis 6, **dadurch gekennzeichnet, daß** die hautkosmetischen und dermatologischen Zubereitungen ausgewählt sind unter kosmetischen Mitteln zur Reinigung der Haut.

8. Verwendung nach Anspruch 7, **dadurch gekennzeichnet, daß** die kosmetischen Mittel zur Reinigung der Haut ausgewählt sind unter Seifen, Syndets, flüssigen Wasch-, Dusch-, und Badepräparaten.

9. Verwendung nach einem der Ansprüche 1 bis 6, **dadurch gekennzeichnet, daß** die hautkosmetischen und dermatologischen Zubereitungen ausgewählt sind unter kosmetischen Mitteln zur Pflege und zum Schutz der Haut, Nagelpflegemitteln sowie Zubereitungen für die dekorative Kosmetik.

10. Verwendung nach Anspruch 9, **dadurch gekennzeichnet, daß** die hautkosmetischen und dermatologischen Zubereitungen ausgewählt sind unter Hautpflegemitteln, Intimpflegemitteln, Fußpflegemitteln, Lichtschutzmitteln, Repellents, Rasiermitteln, Haarentfernungsmitteln, Antiaknemitteln, Make-up, Maskara, Lippenstiften, Lidschatten, Kajalstiften, Eyelinern, Rouges, Puder und Augenbrauenstiften.

11. Verwendung nach Anspruch 10, **dadurch gekennzeichnet, daß** die Hautpflegemittel ausgewählt sind unter W/O- oder O/W-Hautcremes, Tag- und Nachtcremes, Augencremes, Antifaltencremes, Feuchthaltecremes, Bleichcremes, Vitamincremes, Hautlotionen, Pflegelotionen und Feuchthaltelotionen.

12. Verwendung nach einem der Ansprüche 1 bis 11, **dadurch gekennzeichnet, daß** das Copolymer in Form einer W/O-Emulsion eingesetzt wird.

13. Verwendung nach Anspruch 12, **dadurch gekennzeichnet, daß** das Copolymer in der Emulsion oder Suspension polymerisiert worden ist.

14. Verwendung nach Anspruch 12 oder 13, **dadurch gekennzeichnet, daß** die Ölphase der Emulsion oder Suspension ein kosmetisches Öl umfasst.

15. Hautkosmetische oder dermatologische Zubereitung zur Hautpflage, enthaltend mindestens einen Fettkörper sowie neben üblichen Zusätzen ein kationisches Copolymer nach einem der Ansprüche 1 bis 6, wobei das Monomer (a) unter N-Vinylimidazolen ausgewählt ist.

## Claims

1. The nontherapeutic use of at least one copolymer obtainable by
(i) free-radically initiated copolymerization of a monomer mixture comprising
(a) 1 to 99.99% by weight of at least one monomer chosen from N-vinylimidazoles and diallylamines, if appropriate in partially or completely quaternized form;
(b) 0 to 98.99% by weight of at least one neutral or basic water-soluble monomer which is different from (a);
(c) 0 to 50% by weight of at least one unsaturated acid or unsaturated anhydride,
(d) 0 to 50% by weight of at least one free-radically copolymerizable monomer which is different from (a), (b) or (c); and
(e) 0.01 to 10% by weight of at least one monomer which acts as crosslinker and has at least two ethylenically unsaturated, nonconjugated double bonds; and
(ii)subsequent partial or complete quaternization and protonation of the polymer in the case where the monomer (a) is unquaternized or only partially quaternized. as skincare additive for skin cosmetic and dermatological preparations.

2. The use according to claim 1, wherein the amount of monomer (c) is 0 to 40% by weight.

3. The use according to claim 1 or 2, wherein the protonation as in (ii) takes place during formulation of the preparation.

4. The use according to any of claims 1 to 3, wherein monomer (a) is at least one diallylamine derivative of the formula (II), 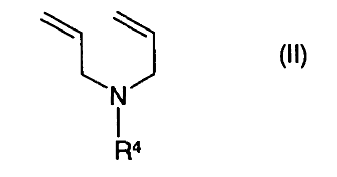 in which the radical R⁴ is C₁-C₂₄ alkyl.

5. The use according to any of claims 1 to 3, wherein monomer (a) is at least one N-vinylimidazole derivative of the formula (I), 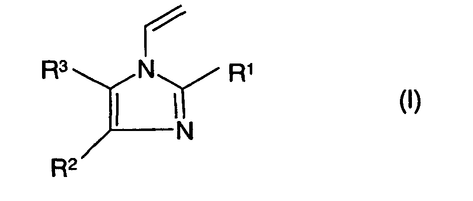 in which the radicals R¹ to R³ independently of one another are hydrogen, C₁-C₄-alkyl or phenyl.

6. The use according to any of claims 1 to 5, wherein monomer (b) is at least one N-vinyllactam.

7. The use according to any of claims 1 to 6, wherein the skin cosmetic and dermatological preparations are chosen from cosmetic compositions for cleansing the skin.

8. The use according to claim 7, wherein the cosmetic compositions for cleansing the skin are chosen from soaps, syndets, liquid washing, shower and bath preparations.

9. The use according to any of claims 1 to 6, wherein the skin cosmetic and dermatological preparations are chosen from cosmetic compositions for the care and protection of the skin, nailcare compositions, and preparations for decorative cosmetics.

10. The use according to claim 9, wherein the skin cosmetic and dermatological preparations are chosen from skincare compositions, personal hygiene care compositions, footcare compositions, sunscreens, repellents, shaving compositions, depilatories, anti-acne compositions, makeup, mascara, lipsticks, eyeshadows, kohl pencils, eyeliners, blushers, powders and eyebrow pencils.

11. The use according to claim 10, wherein the skincare compositions are chosen from W/O or O/W skin creams, day and night creams, eye creams, antiwrinkle creams, moisturizers, bleaching creams, vitamine creams, skin lotions, care lotions and moisturizing lotions.

12. The use according to any of claims 1 to 11, wherein the copolymer is used in the form of a W/O emulsion.

13. The use according to claim 12, wherein the copolymer has been polymerized in the emulsion or suspension.

14. The use according to claim 12 or 13, wherein the oil phase of the emulsion or suspension comprises a cosmetic oil.

15. A skin cosmetic or dermatological skincare preparation comprising at least one fatty substance and, in addition to customary additives, a cationic copolymer according to any of claims 1 to 6, where the monomer (a) is chosen from N-vinylimidazoles.

## Revendications

1. Utilisation non thérapeutique d'au moins un copolymère qui peut être obtenu
(i) par copolymérisation amorcée par voie radicalaire d'un mélange de monomères à base
(a) de 1 à 99,99 % en poids d'au moins un monomère choisi parmi des N-vinylimidazoles et des diallylamines, éventuellement sous une forme partiellement ou totalement quaternisée,
(b) de 0 à 98,99 % en poids d'au moins un monomère différent de (a), soluble dans l'eau, neutre ou basique,
(c) de 0 à 50 % en poids d'au moins un acide insaturé ou un anhydride insaturé,
(d) de 0 à 50 % en poids d'au moins un monomère copolymérisable par voie radicalaire, différent de (a), (b) ou (c), et
(e) de 0,01 à 10 % en poids d'au moins un monomère agissant comme agent de réticulation qui comprend au moins deux doubles liaisons non conjuguées, éthyléniquement insaturées, et
(ii) ensuite par protonation ou quaternisation partielle ou totale du polymère dans le cas où le monomère (a) n'est pas quaternisé ou uniquement partiellement,
comme additif de soin de la peau pour des compositions dermocosmétiques et dermatologiques.

2. Utilisation suivant la revendication 1, **caractérisée en ce que** la quantité de monomère (c) est de 0 à 40 % en poids.

3. Utilisation suivant la revendication 1 ou 2, **caractérisée en ce que** la protonation selon (ii) a lieu lors de la formulation de la composition.

4. Utilisation suivant l'une des revendications 1 à 3, **caractérisée en ce que**, comme monomère (a), on utilise au moins un dérivé de diallylamine de la formule générale (II) : 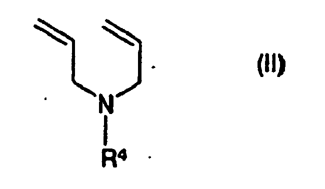 dans laquelle le radical R⁴ représente un groupe alkyle en C₁-C₂₄.

5. Utilisation suivant l'une des revendications 1 à 3, **caractérisée en ce que**, comme monomère (a), on utilise au moins un dérivé de N-vinylimidazole de la formule générale (I) : 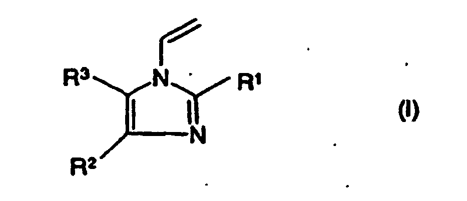 dans laquelle les radicaux R¹ à R³ représentent, indépendamment l'un de l'autre, de l'hydrogène ou un groupe alkyle en C₁-C₄ ou phényle.

6. Utilisation suivant l'une des revendications 1 à 5, **caractérisée en ce que**, comme monomère (b), on utilise au moins un N-vinyllactame.

7. Utilisation suivant l'une des revendications 1 à 6, **caractérisée en ce que** les compositions dermocosmétiques et dermatologiques sont choisies parmi des produits cosmétiques de nettoyage de la peau.

8. Utilisation suivant la revendication 7, **caractérisée en ce que** les produits cosmétiques de nettoyage de la peau sont choisis parmi des savons, des détersifs synthétiques, des préparations de lavage, de douche et de bain liquides.

9. Utilisation suivant l'une des revendications 1 à 6, **caractérisée en ce que** les compositions dermocosmétiques et dermatologiques sont choisies parmi des produits cosmétiques destinés au soin et à la protection de la peau, des produits d'entretien des ongles ainsi que des compositions pour la cosmétique décorative.

10. Utilisation suivant la revendication 9, **caractérisée en ce que** les compositions dermocosmétiques et dermatologiques sont choisies parmi des produits d'entretien de la peau, des produits de soin intime, des produits d'entretien des pieds, des produits de protection contre la lumière, des insectifuges, des produits de rasage, des produits d'épilation, des produits contre l'acné, des maquillages, des mascaras, des rouges à lèvres, des ombres à paupières, des crayons de khôl, des eye-liners, des rouges, des poudres et des crayons à sourcils.

11. Utilisation suivant la revendication 10, **caractérisée en ce que** les produits de soin pour la peau sont choisis parmi des crèmes pour la peau eau-dans-huile ou huile-dans-eau, des crèmes de jour et de nuit, des crèmes pour les yeux, des crèmes antirides, des crèmes hydratantes, des crèmes décolorantes, des crèmes vitaminées, des lotions pour la peau, des lotions de soins et des lotions hydratantes.

12. Utilisation suivant l'une des revendications 1 à 11, **caractérisée en ce que** le copolymère est mis en oeuvre sous la forme d'une émulsion eau-dans-huile.

13. Utilisation suivant la revendication 12, **caractérisée en ce que** le copolymère a été polymérisé en émulsion ou suspension.

14. Utilisation suivant la revendication 12 ou 13, **caractérisée en ce que** la phase huileuse de l'émulsion ou de la suspension comporte une huile cosmétique.

15. Composition dermocosmétique ou dermatologique pour les soins de la peau, contenant au moins un corps gras ainsi que, à côté d'additifs usuels, un copolymère cationique suivant l'une des revendications 1 à 6, le monomère (a) étant choisi parmi des N-vinylimidazoles.
